# EUROPEAN PATENT SPECIFICATION

(11) **EP 4 313 274 B1**
(45) Date of publication and mention of the grant of the patent: **16.09.2026**
(21) Application number: 22714744.4
(22) Date of filing: 10.03.2022
(51) Int. Cl.: A61N 1/372, A61N 1/36, A61N 1/37

(54) **AUTOMATIC CALIBRATION IN AN IMPLANTABLE STIMULATOR DEVICE HAVING NEURAL SENSING CAPABILITY**
AUTOMATISCHE KALIBRIERUNG IN EINER IMPLANTIERBAREN STIMULATORVORRICHTUNG MIT NEURONALER ERFASSUNGSFÄHIGKEIT
ÉTALONNAGE AUTOMATIQUE DANS UN DISPOSITIF DE STIMULATION IMPLANTABLE AYANT UNE CAPACITÉ DE DÉTECTION NEURONALE

(30) Priority: 25.03.2021 US 202163165825 P
(43) Date of publication of application: 07.02.2024
(73) Proprietor: Boston Scientific Neuromodulation Corporation, Valencia, CA 91355 (US)
(72) Inventor: ESTELLER, Rosana, Santa Clarita, CA 91390 (US); LARCOM, Jonathan, Simi Valley, CA 93065 (US)
(74) Representative: Vossius & Partner Patentanwälte Rechtsanwälte mbB
(86) International application number: PCT/US2022/071073
(87) International publication number: WO 2022/204650

(56) References cited:
- US-A1- 2019 209 844
- US-A1- 2020 147 397

## Description

### FIELD OF THE INVENTION

This application relates to Implantable Medical Devices (IMDs), and more specifically to circuitry to assist with calibrating stimulation in an implantable stimulator device.

### INTRODUCTION

Implantable neurostimulator devices are devices that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will generally focus on the use of the invention within a Spinal Cord Stimulation (SCS) system, such as that disclosed in U.S. Patent 6,516,227. However, the present invention may find applicability with any implantable neurostimulator device system.

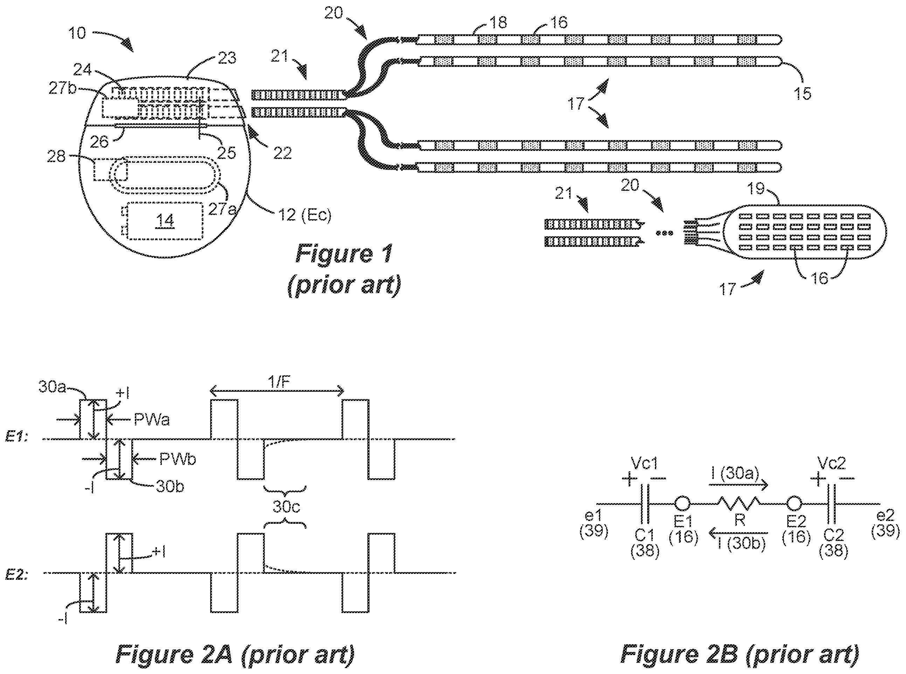

An SCS system typically includes an Implantable Pulse Generator (IPG) 10 shown in Figure 1. The IPG 10 includes a biocompatible device case 12 that holds the circuitry and a battery 14 for providing power for the IPG to function. The IPG 10 is coupled to tissue-stimulating electrodes 16 via one or more electrode leads that form an electrode array 17. For example, one or more percutaneous leads 15 can be used having ring-shaped or split-ring electrodes 16 carried on a flexible body 18. In another example, a paddle lead 19 provides electrodes 16 positioned on one of its generally flat surfaces. Lead wires 20 within the leads are coupled to the electrodes 16 and to proximal contacts 21 insertable into lead connectors 22 fixed in a header 23 on the IPG 10, which header can comprise an epoxy for example. Once inserted, the proximal contacts 21 connect to header contacts 24 within the lead connectors 22, which are in turn coupled by feedthrough pins 25 through a case feedthrough 26 to stimulation circuitry 28 within the case 12.

In the illustrated IPG 10, there are thirty-two electrodes (E1-E32), split between four percutaneous leads 15, or contained on a single paddle lead 19, and thus the header 23 may include a 2x2 array of eight-electrode lead connectors 22. However, the type and number of leads, and the number of electrodes, in an IPG is application specific and therefore can vary. The conductive case 12 can also comprise an electrode (Ec). In a SCS application, the electrode lead(s) are typically implanted in the spinal column proximate to the dura in a patient's spinal cord, preferably spanning left and right of the patient's spinal column. The proximal contacts 21 are tunneled through the patient's tissue to a distant location such as the buttocks where the IPG case 12 is implanted, at which point they are coupled to the lead connectors 22. In other IPG examples designed for implantation directly at a site requiring stimulation, the IPG can be lead-less, having electrodes 16 instead appearing on the body of the IPG 10 for contacting the patient's tissue. The IPG lead(s) can be integrated with and permanently connected to the IPG 10 in other solutions. The goal of SCS therapy is to provide electrical stimulation from the electrodes 16 to alleviate a patient's symptoms, such as chronic back pain.

IPG 10 can include an antenna 27a allowing it to communicate bi-directionally with a number of external devices discussed subsequently. Antenna 27a as shown comprises a conductive coil within the case 12, although the coil antenna 27a can also appear in the header 23. When antenna 27a is configured as a coil, communication with external devices preferably occurs using near-field magnetic induction. IPG 10 may also include a Radio-Frequency (RF) antenna 27b. In Figure 1, RF antenna 27b is shown within the header 23, but it may also be within the case 12. RF antenna 27b may comprise a patch, slot, or wire, and may operate as a monopole or dipole. RF antenna 27b preferably communicates using far-field electromagnetic waves, and may operate in accordance with any number of known RF communication standards, such as Bluetooth, Zigbee, WiFi, MICS, and the like.

Stimulation in IPG 10 is typically provided by pulses each of which may include a number of phases such as 30a and 30b, as shown in the example of Figure 2A. Stimulation parameters typically include amplitude (current I, although a voltage amplitude V can also be used); frequency (F); pulse width (PW) of the pulses or of its individual phases such as 30a and 30b; the electrodes 16 selected to provide the stimulation; and the polarity of such selected electrodes, i.e., whether they act as anodes that source current to the tissue or cathodes that sink current from the tissue. These and possibly other stimulation parameters taken together comprise a stimulation program that the stimulation circuitry 28 in the IPG 10 can execute to provide therapeutic stimulation to a patient.

In the example of Figure 2A, electrode E1 has been selected as an anode (during its first phase 30a), and thus provides pulses which source a positive current of amplitude +I to the tissue. Electrode E2 has been selected as a cathode (again during first phase 30a), and thus provides pulses which sink a corresponding negative current of amplitude -I from the tissue. This is an example of bipolar stimulation, in which only two lead-based electrodes are used to provide stimulation to the tissue (one anode, one cathode). However, more than one electrode may be selected to act as an anode at a given time, and more than one electrode may be selected to act as a cathode at a given time.

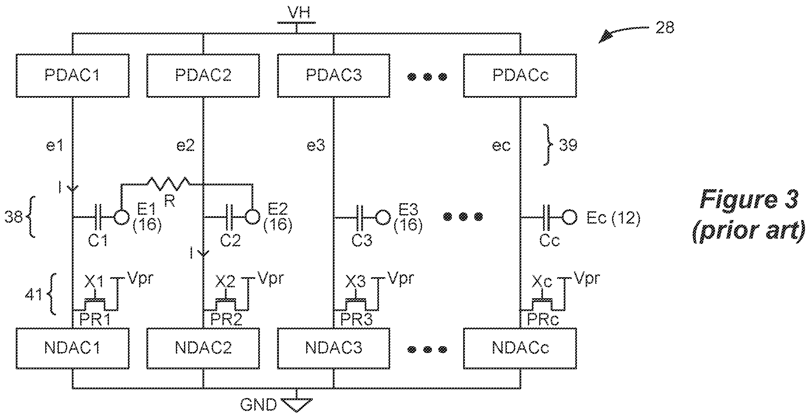

IPG 10 as mentioned includes stimulation circuitry 28 to form prescribed stimulation at a patient's tissue. Figure 3 shows an example of stimulation circuitry 28, which includes one or more current source circuits and one or more current sink circuits. The sources and sinks can comprise Digital-to-Analog converters (DACs), and may be referred to as PDACs and NDACs in accordance with the Positive (sourced, anodic) and Negative (sunk, cathodic) currents they respectively issue. In the example shown, a NDACi/PDACi pair is dedicated (hardwired) to a particular electrode node ei 39. Each electrode node ei 39 is connected to an electrode Ei 16 via a DC-blocking capacitor Ci 38, for the reasons explained below. The stimulation circuitry 28 in this example also supports selection of the conductive case 12 as an electrode (Ec 12), which case electrode is typically selected for monopolar stimulation. The PDACs and NDACs can also comprise voltage sources.

Proper control of the PDACs and NDACs allows any of the electrodes 16 to act as anodes or cathodes to create a current through a patient's tissue, R, hopefully with good therapeutic effect. In the example shown, electrode E1 has been selected as an anode electrode to source current to the tissue R and E2 as a cathode electrode to sink current from the tissue R. Thus PDAC1 and NDAC2 are activated and digitally programmed to produce the desired current, I, with the correct timing (e.g., in accordance with the prescribed frequency F and pulse widths PWa and PWb). Power for the stimulation circuitry 28 is provided by a compliance voltage VH, as described in further detail in U.S. Patent Application Publication 2013/0289665. As shown the compliance voltage may be coupled to the source circuitry (e.g., the PDAC(s)), while ground may be coupled to the sink circuitry (e.g., the NDAC(s)), such that the stimulation circuitry is coupled to and powered between the compliance voltage and ground. More than one anode electrode and more than one cathode electrode may be selected at one time, and thus current can flow through the tissue R between two or more of the electrodes 16. Stimulation circuitry 28 can differ. In an example not shown, a switching matrix can intervene between the one or more PDACs and the electrode nodes ei 39, and between the one or more NDACs and the electrode nodes, to allow any of the PDACs or NDACs to be connected to any of the electrode nodes. Various examples of stimulation circuitries can be found in USPs 6,181,969, 8,606,362, 8,620,436, 10,912,942, and U.S. Patent Application Publication 2018/0071520. Much of the stimulation circuitry 28 of Figure 3, including the PDACs and NDACs, the switch matrices (if present), and the electrode nodes ei 39 can be integrated on one or more Application Specific Integrated Circuits (ASICs), which may also contain other circuitry useful in the IPG 10, such as telemetry circuitry (for interfacing off chip with telemetry antennas 27a and/or 27b), circuitry for generating the compliance voltage VH, various measurement circuits, etc.

Also shown in Figure 3 are DC-blocking capacitors Ci 38 placed in series in the electrode current paths between each of the electrode nodes ei 39 and the electrodes Ei 16 (including the case electrode Ec 12). The DC-blocking capacitors 38 act as a safety measure to prevent DC current injection into the patient, as could occur for example if there is a circuit fault in the stimulation circuitry 28, and also generally comprise part of the IPG's charge balancing mechanism. The DC-blocking capacitors 38 are typically provided off-chip (off of the ASIC(s)), and instead may be provided in or on a circuit board in the IPG 10 used to integrate its various components, as explained in U.S. Patent Application Publication 2015/0157861.

Referring again to Figure 2A, the stimulation pulses as shown are biphasic, with each pulse comprising a first phase 30a followed thereafter by a second phase 30b of opposite polarity. Biphasic pulses are useful to actively recover any charge that might be stored on capacitive elements in the electrode current paths, such as on the DC-blocking capacitors 38. Charge recovery is shown with reference to both Figures 2A and 2B. During the first pulse phase 30a, charge will (primarily) build up across the DC-blockings capacitors C1 and C2 associated with the electrodes E1 and E2 used to produce the current, giving rise to voltages Vc1 and Vc2 (I = C * dV/dt). During the second pulse phase 30b, when the polarity of the current I is reversed at the selected electrodes E1 and E2, the stored charge on capacitors C1 and C2 is recovered, and thus voltages Vc1 and Vc2 hopefully return to 0V at the end the second pulse phase 30b. To recover all charge by the end of the second pulse phase 30b of each pulse (Vc1 = Vc2 = 0V), the first and second phases 30a and 30b are charged balanced at each electrode, with the phases comprising an equal amount of charge but of the opposite polarity. Passive charge recovery can also occur after the active pulse phases such as 30a and 30b. This can occur during periods 30c, when passive charge recovery switches 41 (Fig. 3) are closed. Passive charge recovery is explained further in USPs 10,716,937 and 10,792,491.

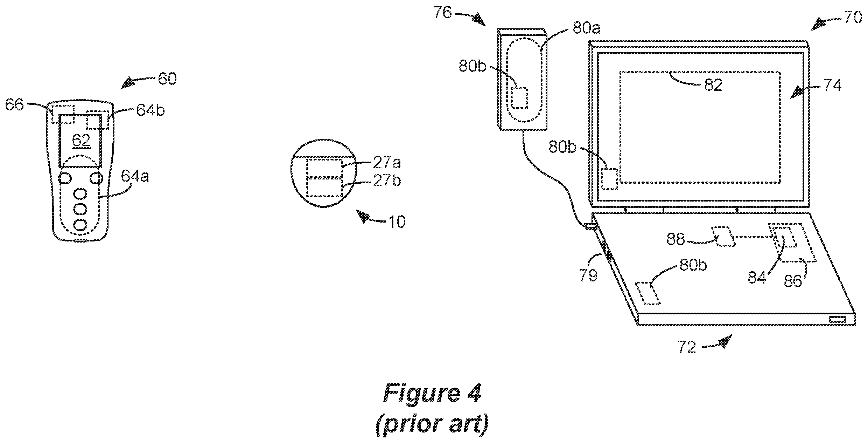

Figure 4 shows various external devices that can wirelessly communicate data with the IPG 10, including a patient, hand-held external controller 60, and a clinician programmer 70. Both of devices 60 and 70 can be used to wirelessly transmit a stimulation program to the IPG 10-that is, to program its stimulation circuitry 28 to produce stimulation with desired amplitudes and timings as described earlier. Both devices 60 and 70 may also be used to adjust one or more stimulation parameters of a stimulation program that the IPG 10 is currently executing. Devices 60 and 70 may also wirelessly receive information from the IPG 10, such as various status information, etc. Devices 60 and 70 may additionally communicate with an External Trial Stimulator (ETS) which is used to mimic operation of the IPG 10 during a trial period and prior to the IPG's implantation, as explained in USPs 9,724,508 and 9,259,574.

External controller 60 can be as described in U.S. Patent Application Publication 2015/0080982 for example, and may comprise a controller dedicated to work with the IPG 10. External controller 60 may also comprise a general purpose mobile electronics device such as a mobile phone which has been programmed with a Medical Device Application (MDA) allowing it to work as a wireless controller for the IPG 10, as described in U.S. Patent Application Publication 2015/0231402. External controller 60 includes a user interface, preferably including means for entering commands (e.g., buttons or selectable graphical icons) and a display 62. The external controller 60's user interface enables a patient to adjust stimulation parameters, although it may have limited functionality when compared to the more-powerful clinician programmer 70, described shortly. The external controller 60 can have one or more antennas capable of communicating with the IPG 10. For example, the external controller 60 can have a near-field magnetic-induction coil antenna 64a capable of wirelessly communicating with the coil antenna 27a in the IPG 10. The external controller 60 can also have a far-field RF antenna 64b capable of wirelessly communicating with the RF antenna 27b in the IPG 10.

Clinician programmer 70 is described further in U.S. Patent Application Publication 2015/0360038, and can comprise a computing device 72, such as a desktop, laptop, or notebook computer, a tablet, a mobile smart phone, a Personal Data Assistant (PDA)-type mobile computing device, etc. In Figure 4, computing device 72 is shown as a laptop computer that includes typical computer user interface means such as a screen 74, a mouse, a keyboard, speakers, a stylus, a printer, etc., not all of which are shown for convenience. Also shown in Figure 4 are accessory devices for the clinician programmer 70 that are usually specific to its operation as a stimulation controller, such as a communication "wand" 76 coupleable to suitable ports on the computing device 72, such as USB ports 79 for example. The antenna used in the clinician programmer 70 to communicate with the IPG 10 can depend on the type of antennas included in the IPG 10. If the patient's IPG 10 includes a coil antenna 27a, wand 76 can likewise include a coil antenna 80a to establish near-filed magnetic-induction communications at small distances. In this instance, the wand 76 may be affixed in close proximity to the patient, such as by placing the wand 76 in a belt or holster wearable by the patient and proximate to the patient's IPG 10. If the IPG 10 includes an RF antenna 27b, the wand 76, the computing device 72, or both, can likewise include an RF antenna 80b to establish communication with the IPG 10 at larger distances. The clinician programmer 70 can also communicate with other devices and networks, such as the Internet, either wirelessly or via a wired link provided at an Ethernet or network port.

To program stimulation programs or parameters for the IPG 10, the clinician interfaces with a clinician programmer graphical user interface (GUI) 82 provided on the display 74 of the computing device 72. As one skilled in the art understands, the GUI 82 can be rendered by execution of clinician programmer software 84 stored in the computing device 72, which software may be stored in the device's non-volatile memory 86. Execution of the clinician programmer software 84 in the computing device 72 can be facilitated by control circuitry 88 such as one or more microprocessors, microcomputers, FPGAs, DSPs, other digital logic structures, etc., which are capable of executing programs in a computing device, and which may comprise their own memories. Such control circuitry 88, in addition to executing the clinician programmer software 84 and rendering the GUI 82, can also enable communications via antennas 80a or 80b to communicate stimulation parameters chosen through the GUI 82 to the patient's IPG 10.

The user interface of the external controller 60 may provide similar functionality because the external controller 60 can include similar hardware and software programming as the clinician programmer. For example, the external controller 60 includes control circuitry 66 similar to the control circuitry 88 in the clinician programmer 70, and may similarly be programmed with external controller software stored in device memory. US2019209844A1 relates generally to medical device systems, and more particularly to neurostimulator systems operable to measure central or peripheral nervous system potentials in the form of compound action potentials either evoked or not by a controlled stimulus or in the form of local field potentials both of which can be used to adjust stimulation therapy.

### SUMMARY

The invention is defined in claim 1. Any methods disclosed hereinafter do not form part of the scope of the invention, and are mentioned for illustrative purposes only. A method is disclosed for determining one or more first thresholds for therapeutic stimulation provided to a patient by an implantable neurostimulator. The method may comprise: (a) providing the therapeutic stimulation to the patient via the implantable neurostimulator, wherein the therapeutic stimulation comprises a plurality of stimulation parameters; (b) determining a value for a neural response, wherein the neural response is formed in response to the therapeutic stimulation; and (c) determining one or more first thresholds for the therapeutic stimulation using the determined value for the neural response, wherein each first threshold is determined using a first mathematical relationship that models each first threshold as a function of values of the neural response.

In one example, the one or more first thresholds comprise thresholds for one of the stimulation parameters that causes a physiological response in the patient. In one example, the physiological response comprises one or more of paresthesia and discomfort. In one example, the one or more first thresholds comprise physiological thresholds. In one example, the one or more physiological thresholds comprise one or more of a perception threshold and a discomfort threshold. In one example, the one or more first thresholds comprise thresholds for an amplitude of the therapeutic stimulation. In one example, the therapeutic stimulation is provided to the spinal column of the patient, and wherein the neural response comprises an Evoked Compound Action Potential. In one example, the value for the neural response comprises a value of one of the stimulation parameters. In one example, the value for the neural response comprises a minimum value of the one of the stimulation parameters at which the neural response is detectable. In one example, the one of the stimulation parameters comprises an amplitude of the therapeutic stimulation. In one example, the first mathematical relationship that models each first threshold is a linear function of the values of the neural response. In one example, the value for the neural response comprises an extracted neural threshold. In one example, an external device communicates with the implantable neurostimulator. In one example, the value for the neural response is determined in the external device. In one example, the method is initiated at a user interface of the external device. In one example, the first mathematical relationship for each of the first thresholds is stored in the external device. In one example, the one or more first thresholds is determined in the external device. In one example, the method may further comprise transmitting the one or more first thresholds to the implantable neurostimulator or to another external device. In one example, the method may further comprise prior to step (a), providing test stimulation to the patient via the implantable neurostimulator, wherein the test stimulation is provided at a plurality of different test pulse widths. In one example, the method may further comprise determining values for a neural response at each of the test pulse widths, wherein the neural response is formed in response to the test stimulation. In one example, the method may further comprise determining a second mathematical relationship that models values for the neural response as a function of pulse width using the values for the neural response as determined at each of the test pulse widths. In one example, in step (a) the therapeutic stimulation is provided to the patient at a therapeutic pulse width. In one example, in step (b) the value for the neural response is determined using the second mathematical relationship determined at the therapeutic pulse width.

A system is disclosed which may comprise: an external device configured to communicate with an implantable neurostimulator, wherein the external device is configured to: (a) program the implantable neurostimulator to provide therapeutic stimulation to a patient, wherein the therapeutic stimulation comprises a plurality of stimulation parameters; (b) determine a value for a neural response, wherein the neural response is formed in response to the therapeutic stimulation; and (c) determine one or more first thresholds for the therapeutic stimulation using the determined value for the neural response, wherein each first threshold is determined using a first mathematical relationship that models each first threshold as a function of values of the neural response.

In one example, the one or more first thresholds comprise thresholds for one of the stimulation parameters that causes a physiological response in the patient. In one example, the physiological response comprises one or more of paresthesia and discomfort. In one example, the one or more first thresholds comprise physiological thresholds. In one example, the one or more physiological thresholds comprise one or more of a perception threshold and a discomfort threshold. In one example, the one or more first thresholds comprise thresholds for an amplitude of the therapeutic stimulation. In one example, the therapeutic stimulation is provided to the spinal column of the patient, and wherein the neural response comprises an Evoked Compound Action Potential. In one example, the value for the neural response comprises a value of one of the stimulation parameters. In one example, the value for the neural response comprises a minimum value of the one of the stimulation parameters at which the neural response is detectable. In one example, the one of the stimulation parameters comprises an amplitude of the therapeutic stimulation. In one example, the first mathematical relationship that models each first threshold is a linear function of the values of the neural response. In one example, the value for the neural response comprises an extracted neural threshold. In one example, the system may further comprise the implantable neurostimulator. In one example, the implantable neurostimulator is configured to sense the neural response. In one example, the implantable neurostimulator is configured transmit information indicative of the sensed neural response to the external device. In one example, the first mathematical relationship for each of the first thresholds is stored in the external device. In one example, the external device is further configured to transmit the one or more first thresholds to the implantable neurostimulator or to another external device. In one example, the external device is further configured, prior to step (a), to program the implantable neurostimulator to provide test stimulation to the patient via the implantable neurostimulator, wherein the test stimulation is provided at a plurality of different test pulse widths. In one example, the external device is further configured to determinize values for a neural response at each of the test pulse widths, wherein the neural response is formed in response to the test stimulation. In one example, the external device is further configured to determine a second mathematical relationship that models values for the neural response as a function of pulse width using the values for the neural response as determined at each of the test pulse widths. In one example, in step (a) the therapeutic stimulation is provided to the patient at a therapeutic pulse width. In one example, in step (b) the value for the neural response is determined using the second mathematical relationship determined at the therapeutic pulse width.

A non-transitory computer readable medium is disclosed which may comprise instructions executable on an external device configured to communicate with an implantable neurostimulator, wherein the instructions are configured to: (a) render a user interface on the external device to allow a user program the implantable neurostimulator to provide therapeutic stimulation to a patient, wherein the therapeutic stimulation comprises a plurality of stimulation parameters; (b) determine a value for a neural response, wherein the neural response is formed in response to the therapeutic stimulation; and (c) determine one or more first thresholds for the therapeutic stimulation using the determined value for the neural response, wherein each first threshold is determined using a first mathematical relationship that models each first threshold as a function of values of the neural response.

### BRIEF DESCRIPTION OF THE DRAWINGS

Figure 1 shows an Implantable Pulse Generator (IPG), in accordance with the prior art.
Figures 2A and 2B show an example of stimulation pulses producible by the IPG, in accordance with the prior art.
Figure 3 shows stimulation circuitry useable in the IPG, in accordance with the prior art.
Figure 4 shows various external devices capable of communicating with and programming stimulation in an IPG, in accordance with the prior art.
Figure 5 shows an improved IPG having neural response sensing, and the ability to adjust stimulation dependent on such sensing.
Figure 6 shows stimulation producing a neural response, and the sensing of that neural response at at least one electrode of the IPG.
Figure 7 shows different physiological thresholds such as pth and dth, and how they relate to amplitude I of stimulation.
Figure 8 shows a Graphical User Interface (GUI) of an external device such as a clinician programmer, and use of the interface to set stimulation for a patient and to determine physiological thresholds for that stimulation.
Figure 9 shows graphs relating physiological thresholds such as pth and dth to extracted neural thresholds (ENTs).
Figure 10 shows an algorithm using measured ENTs to determine physiological thresholds using the relationships of Figure 9.
Figure 11 shows strength-duration curves, and modeling ENTs in accordance with such curves to establish ENTs as a function of duration (pulse width).
Figure 12 shows an algorithm using ENTs measured at different pulse widths to determine physiological thresholds using the relationships of Figure 9 and the ENT modelling of Figure 11.

### DETAILED DESCRIPTION

An increasingly interesting development in pulse generator systems, and in Spinal Cord Stimulator (SCS) pulse generator systems specifically, is the addition of sensing capability to complement the stimulation that such systems provide. For example, and as explained in U.S. Patent Application Publication 2017/0296823, it can be beneficial to sense a neural response in neural tissue that has received stimulation from an SCS pulse generator.

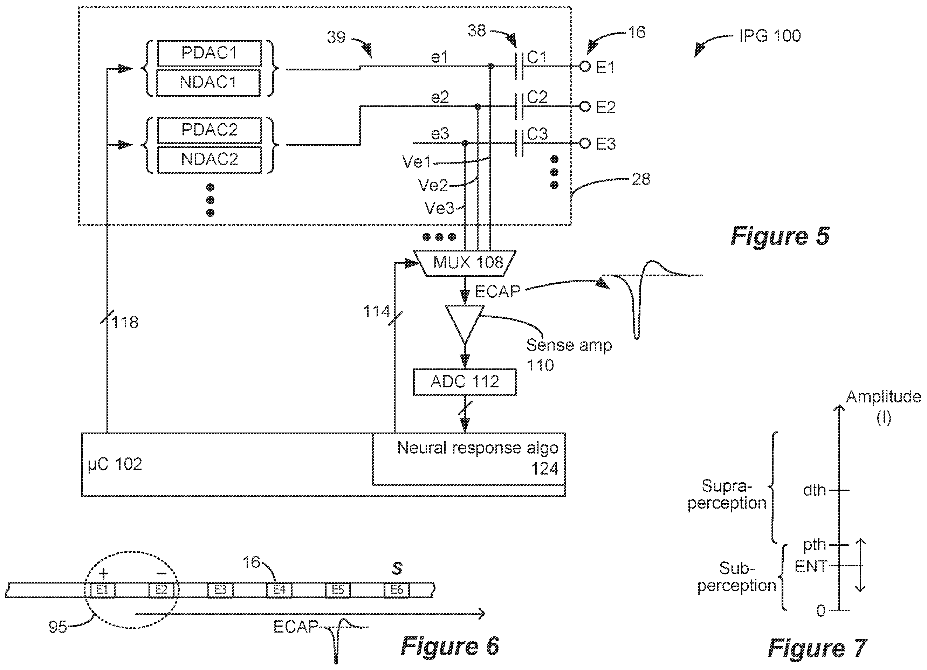

Figure 5 shows circuitry for an SCS IPG 100 having neural response sensing capability. The IPG 100 includes control circuitry 102, which may comprise a microcontroller for example, such as Part Number MSP430, manufactured by Texas Instruments, which is described in data sheets at http://www.ti.com. Other types of control circuitry may be used in lieu of a microcontroller as well, such as microprocessors, FPGAs, DSPs, or combinations of these, etc. Control circuitry 102 may also be formed in whole or in part in one or more Application Specific Integrated Circuits (ASICs) in the IPG 100 as described earlier, which ASIC(s) may additionally include the other circuitry shown in Figure 5.

Figure 5 includes the stimulation circuitry 28 described earlier (Fig. 3), including one or more DACs (PDACs and NDACs). A bus 118 provides digital control signals to the DACs to produce currents or voltages of prescribed amplitudes (I) and with the correct timing (PW, F) at the electrodes selected for stimulation. The electrode current paths to the electrodes 16 include the DC-blocking capacitors 38 described earlier.

The control circuitry 102 is programmed with a neural response algorithm 124 to evaluate a neural response of neurons that fire (are recruited) by the stimulation that the IPG 100 provides. One such neural response depicted in Figures 5 and 6 is an Evoked Compound Action Potential, or "ECAP," although other types of neural responses also exist and can be sensed by the IPG 100. As its name implies, an ECAP comprises a compound (summation) of various action potentials issued from a plurality of recruited neurons, and its amplitude and shape varies depending on the number and type of neural fibers that are firing. Generally speaking, an ECAP can vary between tens of microVolts to tens of milliVolts. The neural response algorithm 124 assesses the ECAP and can, for example, adjust the stimulation program in a closed loop fashion. In this regard, the neural response algorithm 124 can attempt to remove other signals that may be present at the sensing electrode (stimulation artifacts, background signals and noise) and determine one of more features indicative of the size and shape of the ECAP (e.g., peak-to-peak heights, peak areas, line lengths, etc.). Such ECAP features are described in further details in U.S. Patent Application Publication 2020/0305744.

The control circuitry 102 and/or the neural response algorithm 124 can also enable one or more sense electrodes (S) to sense the ECAP, either automatically or based on a user selection of the sense electrode(s) as entered into an external device (see Fig. 4). As shown in Figure 6, the ECAP will be initiated upon stimulation of neural fibers in a recruited neural population 95 proximate to the electrodes chosen for stimulation (e.g., E1 and E2), and will move through the patient's tissue via neural conduction. In the simple example of Figure 6, electrode E6 is chosen as a sense electrode S, and thus this electrode will detect the ECAP as it moves past. The speed at which the ECAP moves depends on the several factors, and is variable.

To assist with selection of the sensing electrode(s), and referring again to Figure 5, each electrode node ei 39 is made coupleable to at least one sense amp 110. In this example, for simplicity, all of the electrode nodes are shown as sharing a single sense amp 110. Thus, any one sensing electrode (e.g., electrode node e6) can be coupled to the sense amp 110 (e.g., Ve6) at a given time per multiplexer 108, as controlled by bus 114. However, although not shown, each electrode node can also be coupleable to its own dedicated sense amp 110. ECAP sensing can also involve differential sensing of the ECAP at more than one electrode (e.g., at electrodes E5 and E6), and thus two electrode nodes (e.g., Ve5 and Ve6) can be input to a differential sense amp 110, as explained for example in U.S. Patent Application Publication 2020/0305744. After the ECAP is sensed, the analog waveform comprising the ECAP is preferably converted to digital signals by an Analog-to-Digital converter 112, which may also reside within the control circuitry 102. The neural response algorithm 124 can then assess the size and shape of the ECAP as already described, and if necessary, make adjustments to stimulation via bus 118. In an alternative, the neural response algorithm 124 may also transmit data to an external device such as the clinician programmer 70 or the patient external controller 60 (Fig. 4), which permits those external systems to perform some of the processing. For example, the algorithm 124 can cause the IPG 100 to transmit digital or analog representations of the waveforms, leaving the external system to determine ECAP features from the waveforms. The algorithm 124 may also cause the IPG 100 to transmit determined ECAP features to the external systems for analysis. In this regard, notice that all or a portion of the neural sensing algorithm 124 can operate in an external system, such as in conjunction with the clinician programmer's software 84 (Fig. 4).

Stimulation in IPG 100 can be provided with reference to a number of different physiological thresholds, which will different from patient to patient. Generally speaking, a physiological threshold comprises a threshold that causes a physiological response in the patient. Reaching a physiological threshold may be perceptible to the patient, such as paresthesia or discomfort. Figure 7 shows an example of two particular physiological thresholds, which are called the perception threshold (pth) and the discomfort threshold (dth). Other physiological thresholds also exist, but are not shown for simplicity. In this example, the physiological thresholds are expressed in terms of current amplitude I of the stimulation therapy that is provided to the patient, although other parameters of the stimulation therapy (e.g., pulse width, frequency) could be used to define these thresholds as well. The perception threshold pth comprises a lowest amplitude at which the patient can still feel the stimulation (as paresthesia), or as a highest amplitude at which the patient cannot feel the stimulation. As such, the perception threshold pth demarks a boundary between sub-perception stimulation therapy (I < pth) and supra-perception stimulation therapy (I > pth). The discomfort threshold dth comprises a highest amplitude at which stimulation is still comfortable for the patient. In other words, stimulation amplitudes above dth (I > dth) are uncomfortable for the patient, and thus these higher amplitudes are generally avoided.

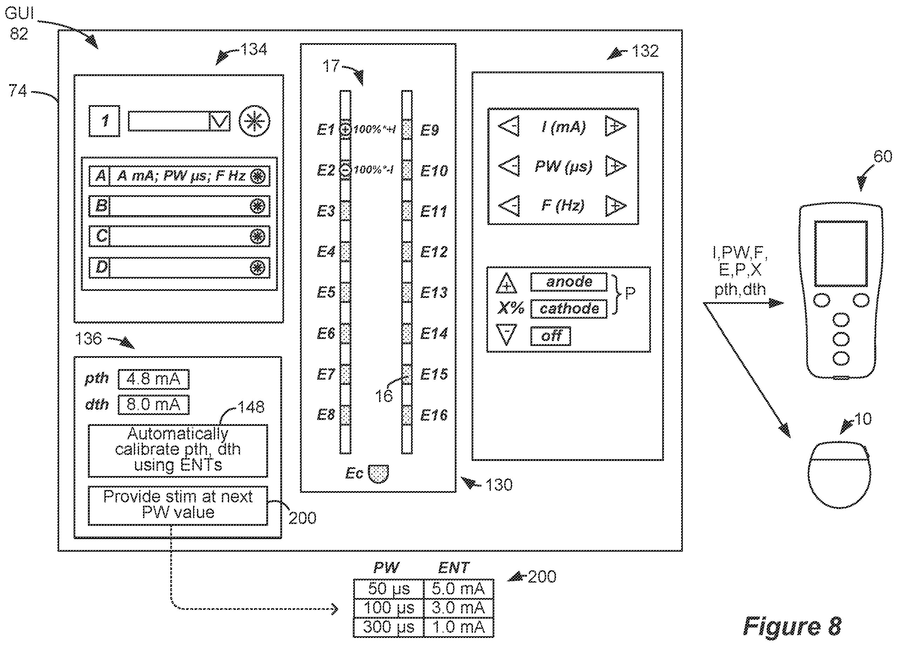

It is normally useful for the clinician to determine at least one of these physiological thresholds for a given patient, because this can be useful to programming or controlling the patient's stimulation therapy. For example, pth can be important to determine if it is desired that a patient receive sub-perception stimulation therapy or supra-perception therapy. dth can be important to determine to ensure that stimulation therapy does not cause patient discomfort. Physiological thresholds can be determined by the clinician using the GUI 82 of the clinician programmer 70 as shown in Figure 8, which can also be used to generally determine optimal stimulation parameters for the patient. The GUI 82 as shown in Figure 8 is fairly basic, and an actual implementation can be more complicated and can provide more options to tailor stimulation and/or to provide active or passive charge recovery schemes, although these more advanced details aren't shown. In this example, the GUI 82 includes a lead interface 130 which shows the electrode array 17 as implanted in the patient. The lead interface 130 can show the relative positions of the leads in the electrode array to each other, and can also show the position of the leads relative to the patient's tissue. For example, although not shown, the locations of the patient's vertebrae can also be displayed in the leads interface 130.

The GUI 82 can also include a stimulation parameters interface 132 which is used to set the stimulation parameters of the stimulation that the patient will receives. This can include means to adjust the amplitude (I), pulse width (PW) and frequency (F) of the stimulation pulses. The GUI can also include means to set the location of stimulation in the electrode array 17. This can involve selecting active electrodes (E), the polarity of those active electrodes (P; anode or cathode), and the percentage of amplitude I (X%) that each active electrode should receive. In this example, electrode E1 has been selected as an anode and E2 as a cathode, with each receiving 100% of current amplitude I as an anodic current (+I) and as a cathodic current (-I). However, and as mentioned earlier, more than one electrode can be selected as an anode and more than one electrode can be selected as a cathode at a given time by sharing the anodic or cathodic current between those anode/cathode electrodes, as dictated by percentage X%. Sharing the anodic and cathodic currents between different numbers of electrodes can set the position of anode and cathode poles (+ and -) as virtual poles between the physical location of the electrodes, as is known. Typically, the location of the stimulation in the electrode array 17 can be manipulated by the clinician, such as by using a computer mouse to move the location of the stimulation within the leads interface 130, and this is done with the goal of locating a position that treats the patient's symptoms (e.g., pain). Note that an electrode configuration algorithm can be used to automictically determine active electrodes (E), polarities (P), and percentages (X%) as the clinician positions the stimulation in the electrode array, as explained further in USP 10,881,859. GUI 82 can also include a program interface 134 to allow a clinician to store and load stimulation programs for the patient.

Once generally optimal stimulation parameters (I, PW, F, E, P, X) have been determined for the patient, the clinician can determine one or more physiological thresholds discussed earlier. This can generally involve an amplitude "sweep" where the amplitude is set to 0 and is gradually increased until the patient first starts to perceive the stimulation (which establishes pth). Further increasing the amplitude until the patient experiences discomfort similarly establishes dth. Once these thresholds have been determined in this manner, they can be stored by the clinician in a threshold interface 136 in the GUI 82 along with the other stimulation parameters, which then allows these physiological thresholds to be used in setting or controlling the patient's stimulation. For example, once dth is set, the GUI 82 may set this as a maximum value for amplitude I, and may limit amplitude I adjustments to values lower than this maximum for patient safety. In another example, if the clinician decides that the patient should receive paresthesia-based therapy (i.e., supra-perception therapy where the patient perceives a sensation produced by the stimulation), the GUI 82 may set pth as an amplitude minimum, while also setting dth as an amplitude maximum for safety. Percentages of these values can be used as well. For example, the maximum amplitude may be set to 90% of dth to ensure some guardband against patient discomfort. Likewise, if the clinician decides that the patient should receive paresthesia-free (sub-perception) therapy, the GUI 82 may set pth as an amplitude maximum. Again, the maximum amplitude may be limited to a percentage of pth, such as 90% of pth to guardband against the possibility that the patient may feel the stimulation. The optimal stimulation parameters and any relevant physiological thresholds such as pth and/or dth can also be transmitted to and stored in the patient's external controller 60 and/or the patient's IPG 100, as shown in Figure 8. Having the physiological thresholds transmitted to such patient devices is useful to control the extent to which a patient can adjust his situation therapy, such as by limiting amplitude adjustments to different treatment regimes (supra-perception, sub-perception, sub-discomfort, etc.) as just described.

While establishing physiological thresholds such as pth and dth can be useful, it does take some time for the clinician to perform. This can create problems for the clinician when trying to determine optimal stimulation parameters for the patient. As noted above, the clinician can attempt to move the location of the stimulation in the electrode array to try and find a location that best treats the patient's symptoms. Typically, the values of physiological thresholds such as pth and dth will change as the location of the stimulation changes. This can mean that the clinician may need to determine these thresholds at each new stimulation location, which as noted takes some time to manually establish.

Another shortcoming to determining pth and dth as described is that these thresholds are typically set once at the beginning of stimulation therapy, and may thereafter only be altered by the clinician from time to time. This is unfortunate, because it may be useful to adjust such thresholds in between clinician visits. In this regard, it is known that it can be necessary to adjust a patient's stimulation, because the stimulation environment has changed. If a patient changes position, such as going from sitting to standing, this can bring the electrodes closer to or farther from the spinal neural tissue. This would suggest that the intensity of stimulation (e.g., amplitude) may need to be decreased or increased to bring about the same therapeutic effect when treating a patient's symptoms. Scar tissue or changes to the electrode/tissue interface may also naturally change over time, which would also suggest that it may be beneficial to adjust a patient's stimulation. It would be expected that such changes to the stimulation environment would suggest the need to adjust the physiological thresholds. For example, if it is necessary to generally increase the amplitude of simulation given such environmental changes, it would be expected that pth and dth should also increase. However, pth and dth as just discussed are typically set or adjusted by the clinician only infrequently, as described above.

It would be beneficial to automatically change or update physiological thresholds such as pth and dth using measurements taken from the patient. This would allow clinician to more quickly establish values for such thresholds, and would allow such thresholds to be adjusted even after leaving a clinician's office. In this disclosure, neural response measurements are used to estimate, adjust, and set therapeutic thresholds. More specifically, extracted neural thresholds (ENTs) are determined. An ENT may be expressed in terms of current amplitude I of the stimulation therapy that is provided to the patient, and comprises the minimum amplitude at which a neural response can be reliably detected, as described further below. The inventors have noticed a correlation between ENTs and physiological thresholds such as pth and dth, which allows such thresholds to be estimated and updated using measured ENT values. In particular, the inventors have noticed a parallel between the strength-duration curves for ENTs and physiological thresholds such as pth and dth, which again allows physiological thresholds to be estimated and updated using measured ENT values. This is beneficial, because ENTs can be objectively measured, which allows physiological thresholds to be automatically and quickly adjusted on the fly. This both assists the clinician in determining physiological thresholds, and also allows for updating of these thresholds without a clinician's assistance.

An extracted neural threshold (ENT) as just noted may be expressed in terms of current amplitude I of the stimulation therapy that is provided to the patient, as shown in Figure 7, and comprises the smallest amplitude at which an neural response to the stimulation can be reliably detected. One such neural response comprises an ECAP, which as mentioned above is a small signal which may be difficult to detect. The ability to detect an ECAP-and thus determine an ENT value (in mA)-depends on many factors, such as the design of the sense amp(s) 110 (Fig. 5), the resolution with which the sensed ECAP is sampled (via ADC 112), and the particulars of the neural response algorithm 124 (which as noted above can operate at least in part in external systems as well). Furthermore, sensing an ECAP is made difficult because the voltage in a patient's tissue can vary, both as a result of the stimulation and background noise in the tissue. See, e.g., U.S. Patent Application Publications 2020/0305744 and PCT (Int'l) Publication WO 2020/251899. Still further, the sensing electrode(s) used to sense ECAPs may not be constant with respect to stimulation provided in different patients; the distance between the stimulating and sensing electrodes may vary for example. Adding further variability is the fact that the ECAPs can be detected in different ways. For example, an ENT can be determined by the neural response algorithm 124 using signal averaging. See, e.g., USP 10,926,092. An ENT can also be determined "visually"-i.e., as a lowest amplitude at which an ECAP (e.g., its shape) can be visually noticed by a clinician and input into the GUI. (Normally it would be expected that ENT extracted mathematically would be lower than an ENT extracted visually).

For these reasons, an ENT, although measured objectively, does not comprise an absolute value, but instead has a value that may be system and/or patient dependent. In this regard, and referring again to Figure 7, an ENT may have an uncertain relationship to other therapeutic thresholds such as the perception threshold pth. For example, ECAPs are present in sub-perception stimulation therapy, and so one could expect ENTs to be of lower values than pth. However, such ENTs could be higher than pth depending on how ENTs are measured by the system, and depending on the specific patient lead implantation location and its distance to the spinal cord. Note that ECAPs comprise only one type neural response to stimulation, and that other types of neural responses exist for which extracted thresholds can be determined. For example, in Deep Brain Stimulation, stimulation of brain tissue can give rise to Evoked Resonant Neural Activity (ERNA) responses, as explained in U.S. Patent Application Serial No. 17/388,818, filed July 29, 2021. Neural thresholds of still evoked potentials are possible. Note also that ENTs as described here also comprise a type of physiological threshold in that it comprises a threshold (e.g., of current amplitude) that causes a physiological response (a detectable ECAP response) in the patient.

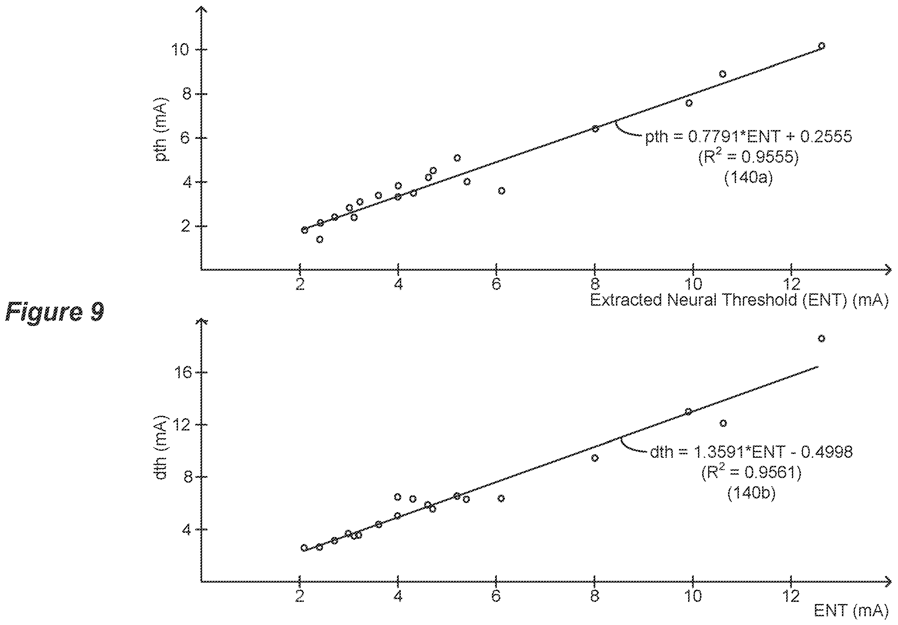

Even though ENT values may have some variability, the inventors have noticed based on empirical measurements that ENTs as measured in a given system vary predictably with physiological thresholds such as pth and dth otherwise determined by the system. This is shown in Figure 9, which shows data taken from different patients. For each patient, an extracted neural threshold (ENT) was measured. Each patient's perception threshold pth (top graph) and discomfort threshold dth (bottom graph) were also determined as described above. As the figures shown, both pth and dth show a significant correlation to ENT. Specifically, using curve fitting (e.g., least squared) techniques, a mathematical relationship 140a between pth and ENT has been determined, and a mathematical relationship 140b between dth and ENT has been determined. Notice that these relationships 140a and 140b do not comprise a mere scalar between ENT as measured and the therapeutic thresholds pth and dth (i.e., pth and dth are not just equal to c * ENT, where c is a constant). In this example, the relationships 140a and 140b are linear, but could be modelled as different functions (e.g., exponentials, logarithms, polynomials, etc.).

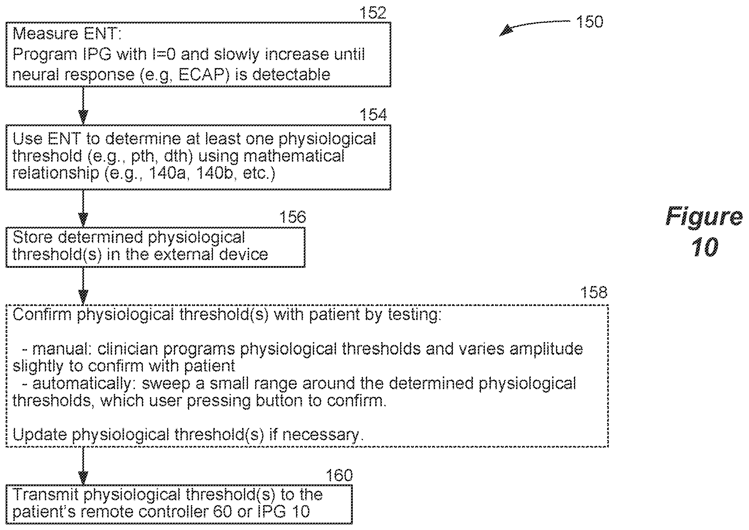

Figure 10 shows a first algorithm 150 which can be used to measure ENTs, and to automatically estimate or calibrate one or more physiological thresholds such as pth or dth using the relationships 140a or 140b. In the example of Figure 10, certain steps in the algorithm 150 are performed by an external device, such as a clinician programmer 70 or patient external controller 60. However, this is not strictly necessary, and instead the entirety of algorithm 150 can be performed within the IPG 100 itself (as programmed in its control circuitry 102), particularly if the relationships 140a or 140b are stored in the IPG 100. It is assumed here that algorithm 150 is initiated by the GUI at option 148 (Fig. 148). Note that aspects of algorithm 150 may be embodied in instructions stored in non-transitory computer readable media such as solid state, optical or magnetic memories, which may be included whole or in part in the external device or the IPG 100. Algorithm 150 may comprise part of the external device's software (e.g., 84, Fig. 4), and may be integrated with the software used to render the GUI 82.

In step 152, an ENT value is measured using stimulation parameters determined earlier for the patient. Specifically, and assuming option 148 is used to start the algorithm 150, the external device causes the IPG 100 to increase the amplitude I starting from zero, until a neural response such as an ECAP is detectable (either using extraction or visualization). The ENT could also be determined by decreasing I until ECAPs are no longer detectable. Because step 152 implicates use of the neural response algorithm 124, and because this algorithm 124 can also operate in part in the external device, step 152 may be performed at least in part in the external device or wholly within the IPG 100. Again, the neural response algorithm 124 can determine the ENT value using extraction or visualization techniques as described earlier.

In step 154, the determined ENT value is used to determine at least one neural threshold, such as pth (using relationship 140a) or dth (using relationship 140b). These relationships may be stored in the external device in conjunction with other aspects of algorithm 150. One skilled will understand that the physiological thresholds can be determined by entering the ENT value into the relationships 140a and 140b and solving for pth and/or dth. Again, if the relationships 140a and 140b are stored in the IPG 100, this step 154 can also be performed entirely within the IPG 100.

In step 156, the physiological thresholds pth and/or dth determined in step 156 are stored in the external device. In particular, the algorithm 150 may automatically populated these determined physiological thresholds into the threshold interface 136 of the GUI (Fig. 8).

At this point, an optional step 158 may be performed to confirm that the determined physiological thresholds are at proper values by testing them on the patient. This is simpler and faster than determining these thresholds as described above using a full amplitude sweep. For example, in a manual mode, the amplitude value is set to the determined pth value (say pth = I = 4.8 mA) and tested on the patient, perhaps by manually moving the amplitude up and down a slight amount from this value. From this, a slightly different value for pth may be determined (e.g., pth = 4.9 mA or 4.7 mA), and this would occur more quickly because a full range of amplitude values is not tested. This example in effect provides an estimated pth value, which can then be quickly updated based on testing. dth may be similarly tested and confirmed. In a more automated approach, a small range of amplitude values is swept around the determined thresholds (e.g., from 4.5 mA to 5.1 mA), with the patient pressing a button (e.g., at I = 4.9 mA) when (in this example) he can first start to feel paresthesia. pth in this example would be calibrated from 4.8 to 4.9 mA.

At step 160, the physiological thresholds as so determined (and perhaps confirmed at step 158) are transmitted to the patient's external controller 60 or to the patient's IPG 10 directly. As noted above, these thresholds can be put to useful ends in controlling patient stimulation therapy. If algorithm 150 runs exclusively in the IPG 100, such transmission of the determined physiological thresholds would not be necessary.

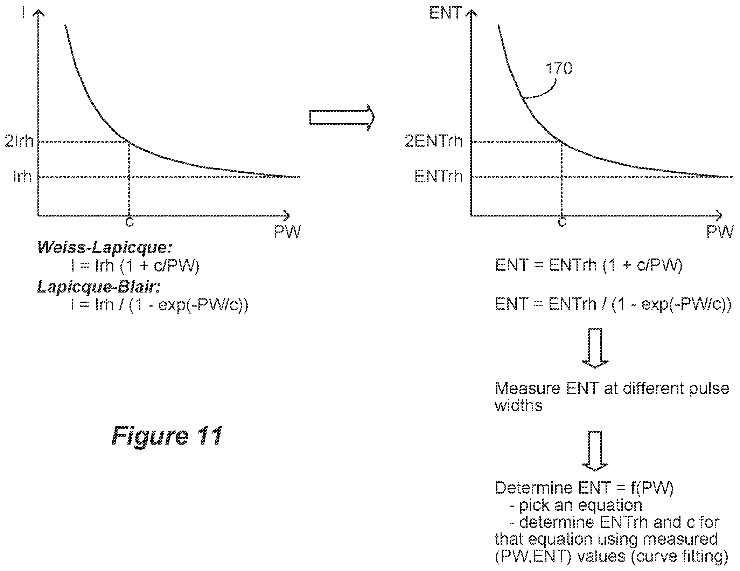

Figure 11 illustrates a manner in which the disclosed technique for determining physiological thresholds using ENT can be extended to include consideration of other stimulation parameters. The left graph in Figure 11 shows a well-known strength-duration curve, which generally shows a relation between strength (e.g., amplitude I) and the duration (e.g., pulse width PW) necessary to recruit neural tissue and generate an action potential. This curve is typically defined by the rheobase (Irb) which generally comprises the minimal current amplitude that results in depolarization, and a chronaxie time (c) which essentially sets the time constant of the displayed curve. This strength-duration curve has been modeled in the literature using different mathematical equations, and two of them (Weiss-Lapicque, and Lapicque-Blair) are shown. ECAPs are the result of multiple action potentials triggered almost at the same time and added together to form the evoked compound action potential. It stands to reason that recruited neural tissue would cause ECAPs to issue, and therefore that ENTs (also measured in intensity or amplitude) would follow these same curves, as shown in the right graph of Figure 11. As such, ENTs can be modelled as a function of duration (pulse width), using the same constants (Irh, c) present in the strength-duration equations.

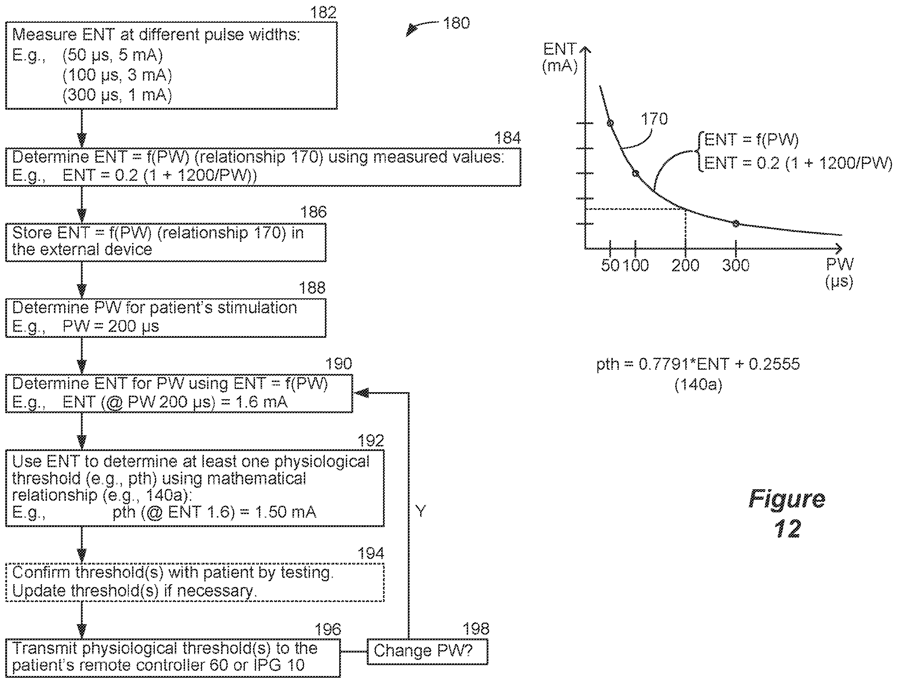

This suggests to the inventor that it may be beneficial to measure ENTs at more than one pulse width. Doing so allows a mathematical relationship 170 to be determined relating ENT values and pulse widths (i.e., ENT = f(PW)). This is shown in further detail in Figure 12, where ENT values have been measured for different pulse widths: (PW, ENT) = (50 µs, 5 mA), (100 µs, 3 mA), and (300 µs, 1 mA). Using the Weiss-Lapicque equation and curve fitting techniques, values for the rheobase (Irh = 0.2 mA) and the chronaxie time (c = 1200 µs) can be determined. In other words, a relationship 170 is determined for the patient which relates ENTs to various pulse widths for their stimulation-i.e., ENT = 0.2 (1 + 1200/PW).

Establishing relationship 170 is useful in the context of the disclosed technique, because it allows physiological thresholds like pth and dth to be estimated for different pulse widths, and algorithm 180 in Figure 12 shows an example of the use of such information. In the example of Figure 12, certain steps in the algorithm 180 are performed by an external device, such as a clinician programmer 70 or patient external controller 60. However, this is not strictly necessary, and instead the entirety of algorithm 180 can be performed within the IPG 100 itself (as programmed in its control circuitry 102), particularly if the relationships 140a, 140b, and 170 are stored in the IPG 100. It is again assumed here that algorithm 180 is initiated by the GUI at option 148 (Fig. 148). Like algorithm 150, algorithm 180 may be embodied in instructions stored in non-transitory computer readable media.

In step 182, an ENT value is measured using stimulation parameters determined earlier for the patient, but with different pulse widths. Specifically, and assuming option 148 is used to start the algorithm 150, the external device may provide an instruction 200 on the GUI 82 instructing the clinician to provide stimulation at a next (first) pulse width value, as shown in Figure 8. This first pule width value (e.g., 50 µs) can be entered using the stimulation parameter interface 132. The clinician can then select instruction 200 to measure the ENT for this pulse width. As before, the external device can cause the IPG 100 to increase the amplitude I until an ECAP is detectable (either using extraction or visualization), or can decreasing I until ECAPs are no longer detectable. The user can then enter a next pulse width to test (e.g., 100 µs), and measure the ENT at this pulse width by again selecting instruction 200, etc. This results in building a data table 200 of (PW, ENT) values, as shown in Figure 8, which data table can be stored in the external device. It should be understood that step 182 essentially involves test stimulation in the sense that such stimulation may involve testing at pulse widths that aren't necessarily therapeutically optimal for the patient.

Step 184 determines the ENT = f(PW) relationship 170 using the data in data table 200. This step was described earlier with respect to Figure 11. Once relationship 170 is determined, it can be stored in the external deice in step 186.

Next is step 188, a therapeutic pulse width to be used for the patient is entered into the GUI (again using stimulation parameters interface 132 for example). In the depicted example, this pulse width value is 200 µs, which is assumed here to be the pulse width that has otherwise been deemed optimal to provide therapeutic stimulation for the patient. While an ENT value could be measured at this optimal pulse width, this is not necessary, because the ENT at PW = 200 µs can be estimated using relationship 170 as just determined, which occurs at step 190. In this example, it is assumed using relationship 170 that ENT = 1.6 mA at PW = 200 µs.

From this estimated ENT value, and at step 192, one or more physiological thresholds like pth or dth can be estimated using the relationship 140a and 140b described earlier. For example purposes, it is only assumed that a single physiological threshold (pth) is determined at step 192. Plugging ENT = 1.6 mA into relationship 140a yields pth = 1.50, which as before can be auto-populated in GUI 82 at threshold interface 136 (Fig. 8). This determined value for pth can be optionally confirmed at step 194, similar to what was described earlier at step 158 in Figure 10, and as before can be transmitted to the patient external controller 60 or 10 to control stimulation to useful effect at step 196.

If later the pulse width of the patient's stimulation is changed (step 196), the physiological threshold(s) can be automatically adjusted without need to take further ENT measurements, because relationships 170, 140a, and/or 140b can be used to adjust the threshold(s). In this regard, and as shown in Figure 12, algorithm 180 can revert back to step 190, where a new ENT value is predicted at the new pulse width using relationship 170, and at step 192 a new physiological threshold(s) (e.g., pth) can be determined using the new ENT value using relationship 140a. In short, algorithm 180 is able to estimate physiological thresholds such as pth and dth using both measured ENT values and pulse width. As was the case earlier, aspects of algorithm 180 can be executed both at the external device and the IPG 100.

To summarize, algorithm 180 (Fig. 12) is more flexible than algorithm 150 (Fig. 10) in that physiological thresholds can be calibrated for a wider range of potential variations in a patient's stimulation program, and in particular when the pule width of the pulses is varied. However, in either case, physiological thresholds are determined automatically, and based on objective ENT measurements, which makes determining these physiological thresholds easier for both the clinician and the patient. It also allows physiological thresholds to be updated without the need for intervention by the clinician. For example, physiological thresholds such as pth and dth can be adjusted on the fly by the patient, or even automatically by the system. In this regard, note that the algorithms 150 can also operate at least in part on a patient's external controller 60. The controller 60 can automatically periodically run either of algorithms 150 or 180 to measure ENTs (possibly at different pulse widths), and to adjust physiological thresholds accordingly, which in turn affects the patient's stimulation. Adjusting dth at the external controller 60 can limit the amplitude I the patient can prescribe, which keeps the patient safe and comfortable. Adjusting pth at the external controller 60 helps to ensure for example that the patient's stimulation will reliably remain sub-perception or supra-perception by constrain the amplitude that the patient can prescribe, etc.

While described in the context of determining physiological thresholds such as pth and dth, it should be understood that the disclosed techniques may also be used to determine target values for stimulation. In this regard, an optimal stimulation amplitude I for a patient can relate to physiological thresholds such as pth and dth. For example, an optimal stimulation amplitude I may comprise pth, a percentage of pth (e.g., I = 70% pth), or a particular value between pth and dth (e.g., a midpoint value such as pth + [dth - pth / 2]). Because physiological thresholds pth and dth can be determined using ENTs as described above, and because a desired amplitude threshold I can be based on or predicted using pth and/or dth, ENTs can be used to predict and/or adjust amplitude I.

## Claims

1. A system, comprising:
an external device (60) configured to communicate with an implantable neurostimulator (100),
wherein the external device is configured to:
(a) program the implantable neurostimulator to provide therapeutic stimulation to a patient, wherein the therapeutic stimulation comprises a plurality of stimulation parameters;
(b) determine a value for a neural response, wherein the neural response is formed in response to the therapeutic stimulation; and **characterised by** the device being further configured to
(c) determine one or more first thresholds for the therapeutic stimulation using the determined value for the neural response, wherein each first threshold is determined using a first mathematical relationship that models each first threshold as a function of values of the neural response.

2. The system of claim 1, wherein the one or more first thresholds comprise thresholds for one of the stimulation parameters that causes a physiological response in the patient, wherein the physiological response comprises one or more of paresthesia and discomfort.

3. The system of claim 1, wherein the one or more first thresholds comprise physiological thresholds, wherein the one or more physiological thresholds comprise one or more of a perception threshold and a discomfort threshold.

4. The system of claim 3, wherein the one or more first thresholds comprise thresholds for an amplitude of the therapeutic stimulation.

5. The system of any of claims 1-4, wherein the therapeutic stimulation is provided to the spinal column of the patient, and wherein the neural response comprises an Evoked Compound Action Potential.

6. The system of any of claims 1-5, wherein the value for the neural response comprises a value of one of the stimulation parameters.

7. The system of claim 6, wherein the value for the neural response comprises a minimum value of the one of the stimulation parameters at which the neural response is detectable, wherein the one of the stimulation parameters comprises an amplitude of the therapeutic stimulation.

8. The system of any of claims 1-7, wherein the first mathematical relationship that models each first threshold is a linear function of the values of the neural response.

9. The system of any of claims 1-8, wherein the value for the neural response comprises an extracted neural threshold.

10. The system of any of claims 1-10, further comprising the implantable neurostimulator, wherein the implantable neurostimulator is configured to sense the neural response.

11. The system of claim 10, wherein the implantable neurostimulator is configured transmit information indicative of the sensed neural response to the external device.

12. The system of claims 10 or 11, wherein the first mathematical relationship for each of the first thresholds is stored in the external device.

13. The system of any of claims 1-12, wherein the external device is further configured, prior to step (a), to program the implantable neurostimulator to provide test stimulation to the patient via the implantable neurostimulator, wherein the test stimulation is provided at a plurality of different test pulse widths.

14. The system of claim 13, wherein the external device is further configured to determinize values for a neural response at each of the test pulse widths, wherein the neural response is formed in response to the test stimulation, wherein the external device is further configured to determine a second mathematical relationship that models values for the neural response as a function of pulse width using the values for the neural response as determined at each of the test pulse widths.

15. A non-transitory computer readable medium comprising instructions executable on an external device configured to communicate with an implantable neurostimulator, wherein the instructions are configured to:
(a) render a user interface on the external device to allow a user program the implantable neurostimulator to provide therapeutic stimulation to a patient, wherein the therapeutic stimulation comprises a plurality of stimulation parameters; **characterised by** said instructions being further configured to
(b) determine a value for a neural response, wherein the neural response is formed in response to the therapeutic stimulation; and
(c) determine one or more first thresholds for the therapeutic stimulation using the determined value for the neural response, wherein each first threshold is determined using a first mathematical relationship that models each first threshold as a function of values of the neural response.

## Patentansprüche

1. System, das aufweist:
eine externe Vorrichtung (60), die so konfiguriert ist, dass sie mit einem implantierbaren Neurostimulator (100) kommuniziert,
wobei die externe Vorrichtung so konfiguriert ist, dass sie:
(a) den implantierbaren Neurostimulator programmiert, um therapeutische Stimulation für einen Patienten bereitzustellen, wobei die therapeutische Stimulation mehrere Stimulationsparameter aufweist;
(b) einen Wert für eine neuronale Reaktion bestimmt, wobei die neuronale Reaktion als Reaktion auf die therapeutische Stimulation erzeugt wird; und
**dadurch gekennzeichnet, dass** die Vorrichtung ferner so konfiguriert ist, dass sie:
(c) einen oder mehrere erste Schwellwerte für die therapeutische Stimulation mit Hilfe des bestimmten Werts für die neuronale Reaktion bestimmt, wobei jeder erste Schwellwert mit Hilfe einer ersten mathematischen Beziehung bestimmt wird, die jeden ersten Schwellwert als Funktion von Werten der neuronalen Reaktion modelliert.

2. System nach Anspruch 1, wobei der eine oder die mehreren ersten Schwellwerte Schwellwerte für einen der Stimulationsparameter aufweisen, der eine physiologische Reaktion im Patienten verursacht, wobei die physiologische Reaktion Parästhesie und/oder Unbehagen aufweist.

3. System nach Anspruch 1, wobei der eine oder die mehreren ersten Schwellwerte physiologische Schwellwerte aufweisen, wobei der eine oder die mehreren physiologischen Schwellwerte einen Wahrnehmungsschwellwert und/oder einen Unbehagenschwellwert aufweisen.

4. System nach Anspruch 3, wobei der eine oder die mehreren ersten Schwellwerte Schwellwerte für eine Amplitude der therapeutischen Stimulation aufweisen.

5. System nach einem der Ansprüche 1 bis 4, wobei die therapeutische Stimulation für die Wirbelsäule des Patienten bereitgestellt wird und wobei die neuronale Reaktion ein evoziertes zusammengesetztes Aktionspotential (Evoked Compound Action Potential) aufweist.

6. System nach einem der Ansprüche 1 bis 5, wobei der Wert für die neuronale Reaktion einen Wert eines der Stimulationsparameter aufweist.

7. System nach Anspruch 6, wobei der Wert für die neuronale Reaktion einen Minimalwert des einen der Stimulationsparameter aufweist, bei dem die neuronale Reaktion detektierbar ist, wobei der eine der Stimulationsparameter eine Amplitude der therapeutischen Stimulation aufweist.

8. System nach einem der Ansprüche 1 bis 7, wobei die erste mathematische Beziehung, die jeden ersten Schwellwert modelliert, eine lineare Funktion der Werte der neuronalen Reaktion ist.

9. System nach einem der Ansprüche 1 bis 8, wobei der Wert für die neuronale Reaktion einen extrahierten neuronalen Schwellwert aufweist.

10. System nach einem der Ansprüche 1 bis 9, das ferner den implantierbaren Neurostimulator aufweist, wobei der implantierbare Neurostimulator so konfiguriert ist, dass er die neuronale Reaktion erfasst.

11. System nach Anspruch 10, wobei der implantierbare Neurostimulator so konfiguriert ist, dass er Informationen über die erfasste neuronale Reaktion zur externen Vorrichtung sendet.

12. System nach Anspruch 10 oder 11, wobei die erste mathematische Beziehung für jeden der ersten Schwellwerte in der externen Vorrichtung gespeichert ist.

13. System nach einem der Ansprüche 1 bis 12, wobei die externe Vorrichtung ferner so konfiguriert ist, dass sie vor dem Schritt (a) den implantierbaren Neurostimulator programmiert, um eine Teststimulation für den Patienten über den implantierbaren Neurostimulator bereitzustellen, wobei die Teststimulation mit mehreren unterschiedlichen Testimpulsbreiten bereitgestellt wird.

14. System nach Anspruch 13, wobei die externe Vorrichtung ferner so konfiguriert ist, dass sie Werte für eine neuronale Reaktion bei jeder der Testimpulsbreiten bestimmt, wobei die neuronale Reaktion als Reaktion auf die Teststimulation erzeugt wird, wobei die externe Vorrichtung ferner so konfiguriert ist, dass sie eine zweite mathematische Beziehung bestimmt, die Werte für die neuronale Reaktion als Funktion der Impulsbreite mit Hilfe der Werte für die neuronale Reaktion modelliert, die bei jeder der Testimpulsbreiten bestimmt werden.

15. Nichttransitorisches computerlesbares Medium mit Befehlen, die in einer externen Vorrichtung ausführbar sind, die zur Kommunikation mit einem implantierbaren Neurostimulator konfiguriert ist, wobei die Befehle so konfiguriert sind, dass sie:
(a) eine Benutzerschnittstelle in der externen Vorrichtung zu befähigen, ein Benutzerprogramm des implantierbaren Neurostimulators zu verwenden, um therapeutische Stimulation für einen Patienten bereitzustellen, wobei die therapeutische Stimulation mehrere Stimulationsparameter aufweist;
**dadurch gekennzeichnet, dass** die Befehle ferner so konfiguriert sind, dass sie
(b) einen Wert für eine neuronale Reaktion bestimmen, wobei die neuronale Reaktion als Reaktion auf die therapeutische Stimulation erzeugt wird; und
(c) einen oder mehrere erste Schwellwerte für die therapeutische Stimulation mit Hilfe des bestimmten Werts für die neuronale Reaktion bestimmen, wobei jeder erste Schwellwert mit Hilfe einer ersten mathematischen Beziehung bestimmt wird, die jeden ersten Schwellwert als Funktion von Werten der neuronalen Reaktion modelliert.

## Revendications

1. Système, comprenant :
un dispositif externe (60) configuré pour communiquer avec un neurostimulateur implantable (100),
dans lequel le dispositif externe est configuré pour :
(a) programmer le neurostimulateur implantable pour qu'il applique une stimulation thérapeutique à un patient, dans lequel la stimulation thérapeutique comprend une pluralité de paramètres de stimulation ;
(b) déterminer une valeur d'une réponse neuronale, dans lequel la réponse neuronale est formée en réponse à la stimulation thérapeutique ; et
**caractérisé en ce que** le dispositif est en outre configuré pour
(c) déterminer un ou plusieurs premiers seuils de la stimulation thérapeutique à l'aide de la valeur déterminée de la réponse neuronale, dans lequel chaque premier seuil est déterminé à l'aide d'une première relation mathématique qui modélise chaque premier seuil en fonction des valeurs de la réponse neuronale.

2. Système selon la revendication 1, dans lequel les un ou plusieurs premiers seuils comprennent des seuils d'un paramètre de stimulation des paramètres de stimulation qui provoque une réponse physiologique chez le patient, dans lequel la réponse physiologique comprend une ou plusieurs parmi une paresthésie et un inconfort.

3. Système selon la revendication 1, dans lequel les un ou plusieurs premiers seuils comprennent des seuils physiologiques, dans lequel les un ou plusieurs seuils physiologiques comprennent un ou plusieurs parmi un seuil de perception et un seuil d'inconfort.

4. Système selon la revendication 3, dans lequel les un ou plusieurs premiers seuils comprennent des seuils d'une amplitude de la stimulation thérapeutique.

5. Système selon l'une des revendications 1 à 4, dans lequel la stimulation thérapeutique est appliquée à la colonne vertébrale du patient, et dans lequel la réponse neuronale comprend un potentiel d'action composé évoqué.

6. Système selon l'une des revendications 1 à 5, dans lequel la valeur de la réponse neuronale comprend une valeur d'un paramètre de stimulation des paramètres de stimulation.

7. Système selon la revendication 6, dans lequel la valeur de la réponse neuronale comprend une valeur minimale de ce paramètre de stimulation des paramètres de stimulation à laquelle la réponse neuronale est détectable, dans lequel ce paramètre de stimulation des paramètres de stimulation comprend une amplitude de la stimulation thérapeutique.

8. Système selon l'une des revendications 1 à 7, dans lequel la première relation mathématique qui modélise chaque premier seuil est une fonction linéaire des valeurs de la réponse neuronale.

9. Système selon l'une des revendications 1 à 8, dans lequel la valeur de la réponse neuronale comprend un seuil neuronal extrait.

10. Système selon l'une des revendications 1 à 9, comprenant en outre le neurostimulateur implantable, dans lequel le neurostimulateur implantable est configuré pour détecter la réponse neuronale.

11. Système selon la revendication 10, dans lequel le neurostimulateur implantable est configuré pour transmettre au dispositif externe des informations indiquant la réponse neuronale détectée.

12. Système selon les revendications 10 ou 11, dans lequel la première relation mathématique pour chacun des premiers seuils est stockée dans le dispositif externe.

13. Système selon l'une des revendications 1 à 12, dans lequel le dispositif externe est en outre configuré avant l'étape (a) pour programmer le neurostimulateur implantable pour qu'il applique une stimulation de test au patient via le neurostimulateur implantable, dans lequel la stimulation de test est appliquée à une pluralité de largeurs d'impulsion de test différentes.

14. Système selon la revendication 13, dans lequel le dispositif externe est en outre configuré pour déterminer des valeurs d'une réponse neuronale à chacune des largeurs d'impulsion de test, dans lequel la réponse neuronale est formée en réponse à la stimulation de test, dans lequel le dispositif externe est en outre configuré pour déterminer une deuxième relation mathématique qui modélise les valeurs de la réponse neuronale en fonction de la largeur d'impulsion à l'aide des valeurs de la réponse neuronale déterminées à chacune des largeurs d'impulsion de test.

15. Support non transitoire lisible par ordinateur comprenant des instructions exécutables sur un dispositif externe configuré pour communiquer avec un neurostimulateur implantable, dans lequel les instructions sont configurées pour :
(a) rendre une interface utilisateur sur le dispositif externe permettant à un utilisateur de programmer le neurostimulateur implantable pour qu'il applique une stimulation thérapeutique à un patient, dans lequel la stimulation thérapeutique comprend une pluralité de paramètres de stimulation ;
**caractérisé en ce que** lesdites instructions sont en outre configurées pour
(b) déterminer une valeur d'une réponse neuronale, dans lequel la réponse neuronale est formée en réponse à la stimulation thérapeutique ; et
(c) déterminer un ou plusieurs premiers seuils de la stimulation thérapeutique à l'aide de la valeur déterminée de la réponse neuronale, dans lequel chaque premier seuil est déterminé à l'aide d'une première relation mathématique qui modélise chaque premier seuil en fonction des valeurs de la réponse neuronale.
